# EUROPEAN PATENT SPECIFICATION

(11) **EP 3 746 010 B1**
(45) Date of publication and mention of the grant of the patent: **05.03.2025**
(21) Application number: 19704395.3
(22) Date of filing: 30.01.2019
(51) Int. Cl.: A61F 5/058

(54) **SPLINTING APPARATUS AND METHOD**
GERÄT UND VERFAHREN ZUR SCHIENUNG
APPAREIL D'ATTELLE ET PROCÉDÉ

(30) Priority: 31.01.2018 GB 201801553
(43) Date of publication of application: 09.12.2020
(73) Proprietor: ARMATREX LTD, Newcastle Upon Tyne, NE20 9NH (GB)
(72) Inventor: BARTON, Derrick Mark Andrew, North Shields Tyne and Wear NE29 6RN (GB)
(74) Representative: Aldridge, Christopher Simon
(86) International application number: PCT/GB2019/050244
(87) International publication number: WO 2019/150094

(56) References cited:
- EP-A2- 2 292 203
- WO-A1-98/46407
- WO-A2-2007/141789
- FR-A5- 2 112 760
- US-A- 3 419 134
- US-A1- 2005 033 207
- US-A1- 2006 079 819

## Description

### TECHNICAL FIELD

The present disclosure relates to a splinting apparatus and particularly, but not exclusively, to a splinting apparatus comprising precursor materials for a curable expandable foam and an expansion chamber into which the foam expands. Aspects of the invention relate to a splinting apparatus, a splinting system and to a method of activating a splint apparatus, where a splinting apparatus, a splinting system and a method of activating a splint apparatus are the subjects of each the independent claims.

### BACKGROUND

Traditionally rigid and semi-rigid inflatable splints are used to immobilise injuries, in particular injuries such as sprains, fractures and breaks. The rigid splint provides support to the injury and prevents the patient from further injury due to movement. Inflatable splints are another splint type used to immobilise injured areas. The splints are inflated by blowing air through a valve once in position around the injury, thus creating a support for the damaged area. A further splint type is a vacuum splint in which the air is evacuated out of a splint resulting in a rigid shape formed by polystyrene balls moulding around the injured area, by doing so, increasing the pressure on the injury compared with alternative splints. Additionally, vacuum splints can be applied to abnormal shaped injuries (as a result of a severe fracture for example), compressing the injury compared to inflatable splints.

Stretchers and head immobilisers are also used to immobilise the spine. Basket stretchers, scoop stretchers and millennia boards are various stretcher types. Basket stretchers are often used for mountain rescue and extracting patients from disaster zones for example. Scoop stretchers are used to move suspected spinal injury patients as they reduce the amount of movement required to place patients on a stretcher so reduce the risk of further injury.

Rigid splints can be uncomfortable for the patient as they are designed to fit a multitude of patients, meaning only a basic moulded shape can be used. Rigid splints and inflatable splints cannot be applied to abnormal shaped injuries such as dislocations or extreme fractures. Inflatable splints may straighten limbs when being inflated which can cause further injury. Further to this, inflatable splints have a risk of bursting, which can result in a sudden loss of support and require a person to manually blow up the splint which can take time. Stretchers are generally inflexible, bulky and heavy making them difficult to transport, particularly in mountain rescues or extraction from natural disasters etc. They also do not conform to an individual shape so often additional blankets are required for comfort and warmth.

Vacuum splints require the manual evacuation of air or a pump to remove the air which can take time and could require extra, heavy equipment to be carried and are prone to failure due to the form being punctured or the valve failing to form an air tight connection and function. Furthermore, the accidental puncture of a vacuum splint will render the splint no longer useable. Vacuum splints also are generally not rigid enough to be used in extraction manoeuvres and are impractical for use in wet or snowy conditions as they can ingest water and/or snow. Vacuum splints can often also have a significantly higher cost compared to rigid splints and stretchers.

Splints made by mixing multiple components to form a foam which then solidifies are known from e.g. US 2005/033207, FR 2112760, US 2006/079819, EP 2292203, WO 2007/141789.

It is an object of embodiments of the invention to at least mitigate one or more of the problems of the prior art.

### SUMMARY OF THE INVENTION

Aspects and embodiments of the invention provide a splinting apparatus and a method of activating a splint apparatus as claimed in the appended claims.

According to a first aspect of the present invention, there is provided a splinting apparatus comprising:
a retaining pouch having a first portion containing a first precursor, a second portion containing a second precursor, and at least one sacrificial seam fluidly separating the first portion from the second portion; and
an expansion chamber enclosing the retaining pouch;
wherein the at least one sacrificial seam is breakable upon application of a force to the retaining pouch to thereby fluidly connect the first and second portions of the retaining pouch such that the first and second precursors mix together, and
wherein mixing of the first and second precursors forms a curable expanded polymer foam.

Suitably, the first portion and the second portion of the retaining pouch are coupled together by the sacrificial seam.

Suitably, each of the first portion and the second portion comprise one of the at least one sacrificial seam.

Suitably, the first and second precursors are miscible.

Suitably, each of the first and second precursors are a monomer precursor.

Suitably, mixing of the first and second precursors provides an exothermic reaction providing heat having a temperature from 20 degrees Celsius to 37 degrees Celsius.

Suitably, the expanded polymer foam is configured to cure within 1 to 9 minutes of breaking of the sacrificial seam.

Suitably, the volume of the expanded polymer foam is about 20 times the total volume of the precursors prior to mixing.

Suitably, the expanded polymer foam is a closed-cell foam.

Suitably, the expanded polymer foam is mouldable prior to curing.

Suitably, each of the first and second portions of the retaining pouch are configured as elongate tubes.

Suitably, the first and second portions extend along from 50% to 90% of the length of the expansion chamber.

Suitably, the retaining pouch is configured to burst upon expansion of the first and second precursors as they mix to form the expanded polymer foam.

The expansion chamber has a volume that is about 110% of the volume of the expanded polymer foam.

Suitably, the apparatus further comprises at least one further retaining pouch.

Suitably, the expansion chamber is formed as a bladder or a bag.

Suitably, the apparatus further comprises an outer sleeve enclosing the expansion chamber.

Suitably, the expansion chamber is secured to an internal surface of the outer sleeve.

Suitably, the outer sleeve comprises an envelope configured to retain the expansion chamber.

Suitably, the envelope comprises an upper layer and a lower layer, and wherein the upper layer and the lower layer are secured together to form the envelope.

Suitably, the outer sleeve is formed from a flexible material.

Suitably, the outer sleeve further comprises securing elements for securing the splinting apparatus around a body part of a patient.

Suitably, the outer sleeve is sized and shaped to form a C-shaped splint.

Suitably, the outer sleeve is configured to further enclose a metal splint for splinting a relatively long limb.

Suitably, the outer sleeve comprises a joining portion for separating first and second envelopes, each of the first and second envelopes retaining an expansion chamber.

According to a second aspect of the present invention, there is provided a splinting system comprising a plurality of splinting apparatus according to the first aspect.

According to a third aspect of the present invention, there is provided a method of activating the splinting apparatus according to the first aspect, the method comprising applying a force to the splinting apparatus to break the sacrificial seam.

Suitably, the step of applying a force comprises twisting the splinting apparatus to provide a torsional force to the splinting apparatus.

Suitably, the method comprises twisting the splinting apparatus in a clockwise and an anti-clockwise direction.

Suitably, the step of applying a force comprises applying a compressional force to the splitting apparatus.

It will be appreciated that any of the methods described herein may be used in combination with any variations of the apparatus described herein.

Certain embodiments have the advantage that the splinting apparatus is X-ray friendly. As such, the splinting apparatus can remain in place to continue to immobilise a limb or other body part during X-ray or a CAT Scan. This can help to alleviate patient discomfort associated with removal of the splinting apparatus prior to X-ray and assist with continued alignment in preparation of remedial repair, in so doing reducing additional pain, discomfort and aggravation of the injury, resulting in swelling and requiring administrating additional analgesia.

Some examples provide a relatively lightweight splinting apparatus compared to previously known splinting apparatus. This can help with transportation of the splinting apparatus, particularly in difficult environments, for example, in mountain rescue operations or remote locations.

Certain embodiments have the advantage that the splinting apparatus is relatively easy to deploy. As such, minimal or no training is needed for a user and the splinting apparatus can be relatively quickly and easily deployed and secured in position.

Certain embodiments are absorbent. As such, any fluids (e.g. bodily fluids, wound exudates, or external fluids) can be absorbed by the apparatus to help keep the patient relatively dry, warm and comfortable. In addition, the medical attendant can also monitor fluids loss as the absorption capacity of the splint is calculated at 10 times its volume for fluid absorption,

Certain embodiments provide the advantage of relatively low heat dissipation during activation. This can help to reduce the risk of delivering excess heat to wound areas.

Some embodiments have the advantage of exhibiting a positive buoyancy. This can be particularly beneficial to help transport patients over water, for example if injured at sea.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

One or more embodiments of the invention will now be described by way of example only, with reference to the accompanying drawings, in which:
Figure 1 shows an example of a splinting apparatus;
Figure 2a shows a further example of a splinting apparatus;
Figure 2b shows the splinting apparatus of Figure 2a in cross-sectional view;
Figure 3a shows a further example of a splinting apparatus;
Figure 3b shows the splinting apparatus of Figure 3a in cross-sectional view;
Figure 4a shows a yet further example of a splinting apparatus;
Figure 4b shows the splinting apparatus of Figure 4a in cross-sectional view;
Figure 4c shows an example method of assembling and packaging a splinting apparatus;
Figure 5a shows an example of a splinting apparatus configured as a stretcher;
Figure 5b shows the splinting apparatus of Figure 5a in cross-sectional view;
Figure 5c shows a side view of a head region of the splinting apparatus of Figure 5a;
Figure 5d shows a cross-sectional view of the head region shown in Figure 5c;
Figure 5e shows an example method of packaging the splinting apparatus in Figure 5a;
Figure 6a to 6f show the stretcher of Figure 5 including a lifting harness arrangement;
Figure 7a shows an example of a splinting apparatus configured as an equine limb splint;
Figure 7b shows the splinting apparatus of Figure 7a in plan view;
Figure 7c shows the splinting apparatus of Figure 7a in cross-sectional view;
Figure 8a shows an example of a splinting apparatus configured as an equine freight blanket;
Figure 8b shows another view of the splinting apparatus of Figure 8a;
Figure 8c shows the splinting apparatus of Figure 8a in plan view;
Figure 9a shows an example of a splinting apparatus tube in a folded state;
Figure 9b shows the splinting apparatus tube in Figure 9a in an unfolded state;
Figure 9c shows the splinting apparatus tube in Figure 9b with support collars; and
Figure 9d shows the splinting apparatus tube in Figure 9c in an activated state.

In the drawings, like reference numerals refer to like parts.

### DETAILED DESCRIPTION

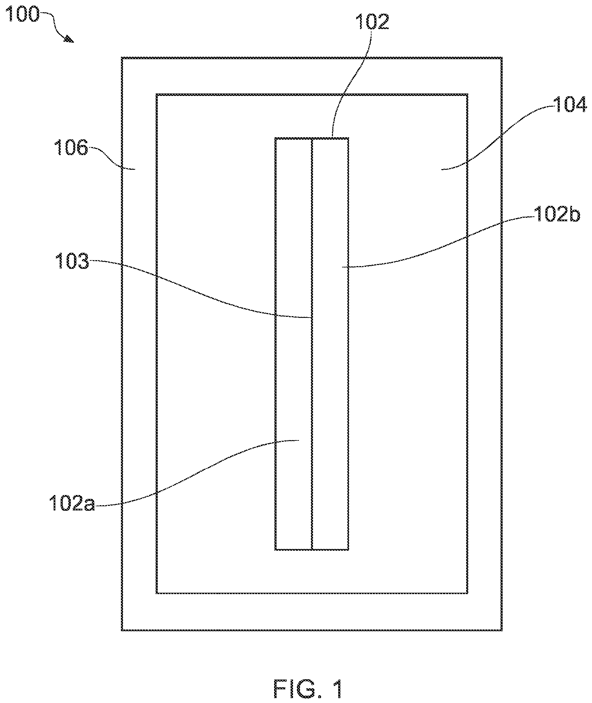

Figure 1 illustrates an example of a splinting apparatus 100. The splinting apparatus 100 includes a retaining pouch 102. The retaining pouch includes a first portion 102a and a second portion 102b. Each of the first and second portions 102a, 102b form an enclosed receptacle for retaining a fluid. The first and second portions 102a, 102b are fluidly separated by a sacrificial seam 103. In this example, the sacrificial seam 103 is configured as a heat weld coupling opposing sides of the retaining pouch 102. As such, the first and second portions 102a, 102b are fluidly separated by the heat weld.

The retaining pouch 102 is aptly formed from a flexible material, for example a polymer film. In this example, each of the first and second portions 102a, 102b are configured as elongate tubes. The elongate tubes are aptly parallel to each other and separated by the sacrificial seam 103.

Each of the first and second portions 102a, 102b contain respective first and second precursors. The first and second precursors are configured such that when mixed together they form a curable expanded polymer foam. The expanded polymer foam is aptly mouldable until cured. Various suitable precursors for forming a foam will be known to those skilled in the art. The precursors are aptly miscible to form a uniform mixture. Each of the first and second precursors may aptly be a monomer precursor.

The first and second precursors are aptly configured to mix to provide an exothermic reaction providing heat having a temperature from 20 degrees Celsius to 37 degrees Celsius. More aptly, the exothermic reaction temperature may be from 30 degrees Celsius to 34 degrees Celsius, or more aptly around 32 degrees Celsius. This helps to provide a warming effect to comfort the patient without overheating the wound or injury area.

One suitable example of the first and second precursors is commercially available Polycraft LD40 expanding Polyurethane Foam. In this example, the first precursor is a resin part and the second precursor is an Isocyanate part. The LD40 foam takes about 140 to 168 seconds to form and the final foam volume is about 20 times the total liquid volume of the first and second precursors. The exothermic reaction temperature of LD40 is about 32 degrees Celsius.

Upon application of a force to the retaining pouch 102, the sacrificial seam 103 is broken to thereby fluidly connect the first and second portions 102a, 102b of the retaining pouch such that the first and second precursors mix together. Aptly, a torsional (twisting) force is applied to the retaining pouch 102 to break the sacrificial seam 103. The torsional force may be applied in a first (e.g. clockwise) direction and then a second (e.g. anticlockwise) direction. The process may be repeated whilst the precursors begin to mix. The torsional forces can help to agitate and mix the first and second precursors to allow for more even expansion of the foam and a more uniform mixture.

Upon mixing, the first and second precursors react together to form a curable expanded polymer foam. As the foam expands, it may burst from the retaining pouch 102, and continue to expand into the surrounding area. In this example, each of the first and seconds portions 102a, 102b contain about 10ml of precursor. The total volume of the expanded polymer foam is aptly about 20 times the total volume of the precursors prior to mixing. As such, in this example, the total volume of the expanded polymer foam may be about 400ml.

An expansion chamber 104 encloses the retaining pouch 102. The expansion chamber 104 contains the expanding foam and helps to guide the expanding foam into the desired areas. In this example, the expansion chamber 104 is substantially rectangular in shape and may be particularly suited to splinting of a limb. The expansion chamber 104 may be any other suitable shape depending on the intended use for the splinting apparatus.

The expansion chamber 104 is configured as a bladder or a bag for enclosing the retaining pouch. In this example, the expansion chamber is formed from a polyester film or plastic sheet. The expansion chamber 104 is preferably formed from a polyester film or plastic sheet of higher gauge than the retaining pouch 102. For example, the expansion chamber may be formed from a film or sheet having a gauge of around 2µm and 2.20 g/m². An example of a suitable polyester film is Mylar^{®} available from DuPont Teijin Films, e.g. 2µm clear Mylar^{®} 2.20 g/m².

The retaining pouch 102 may be attached to the expansion chamber 104. For example, the retaining pouch 102 may be filled with the respective first and second precursors and then glued in place to the inside of the expansion chamber once filled. In other examples, the retaining pouch 102 may simply be placed inside the expansion chamber, without the need for attachment.

The retaining pouch 102 (including the first and second portions) aptly extends along 50% to 90% of the length of the expansion chamber 104, or more aptly 70% to 80% of the length of the expansion chamber. This can help to guide the expanding foam to expand to substantially all areas of the expansion chamber 104.

The expansion chamber is configured to have a volume about 110% of the expected volume of the expanded foam (i.e. the volume to which the foam is expected to expand based on the precursor volume and reaction characteristics). As such, in this example having 10ml of each precursor, with the expanded foam volume expected to be 20 times the volume of the unmixed precursor (i.e. 400ml), the expansion chamber is configured to have a volume of 110% of 400ml, i.e. about 440ml. This allows for extra expansion space in the expansion chamber 104 in case the foam expands to a greater volume than expected, and therefore helps to prevent leakage of the foam in cases of overexpansion.

Whilst the foam is expanding, the splinting apparatus 100 is applied to a patient (e.g. around a patient's broken arm) and secured into position. The expanding foam is mouldable prior to curing and so may conform to the shape of the patient, providing a relatively close fit. The expanded foam is configured to cure from around 1 to 9 minutes after breaking of the sacrificial seam, or more aptly from around 3 to 5 minutes after breaking of the sacrificial seam, or more aptly at around 3 minutes after breaking of the sacrificial seam. This is to allow enough time for a medical practitioner (or first aid attendant) to correctly apply the splinting apparatus to the patient whilst also being time efficient, which can be critical for more serious injuries.

The cured expanded polymer foam is aptly a closed cell foam, which can provide greater strength and stability than an open cell foam. The cured foam is relatively rigid to immobilise the splinted body part. Aptly the cured foam using the Durometer Shore Hardness Scale, has a shore hardness of Shore A 40 or through an Indentation Force Deflection test, is 10mm on 0.4kn [American Society for Testing and Materials - ASTM D3574 Testing for Flexible Cellular Urethane Foams]. The Shore A Hardness Scale measures the hardness of flexible mold rubbers that range in hardness from very soft and flexible, to medium and somewhat flexible, to hard with almost no flexibility at all. Semi-rigid plastics can also be measured on the high end of the Shore A Scale.

Aptly, the cured foam may be a high density straight chain polymer.

In this example, the expansion chamber 104 is enclosed within an outer sleeve 106. The outer sleeve 106 comprises an envelope for retaining the expansion chamber 104. The envelope may comprise an upper layer and a lower layer, which may be stitched together to form a pouch. The envelope may be configured to have a volume of about 120% of the expected volume of the expanded foam. In this way, the outer sleeve 106 can provide further expansion volume to contain the expanding foam in case of overexpansion beyond the volume of the expansion chamber 104. The outer sleeve 106 is aptly stretchable to allow overexpansion of the expansion chamber 104 whilst still being restrained within the envelope of the outer sleeve 106.

The outer sleeve 106 may also include securing elements (e.g. straps, or buckles, or any other releasable strap) for securing the splinting apparatus 100 around a body part of a patient. The securing elements are configured to secure the splinting apparatus in a wrapped configuration around a body part of the patient. In this example, the outer sleeve is sized and shaped to form a substantially C-shaped splint (or gutter shaped splint). With this configuration, the splinting apparatus 100 may be easier to remove as it can be either slipped off after releasing the securing elements, or can be broken or cut away from the body part.

The outer sleeve 106 is aptly formed from a flexible material suitable for wrapping around a body part. The material is aptly relatively lightweight and of suitable gauge to protect the contents (i.e. the expansion chamber and the retaining pouch). In one example, the outer sleeve 106 may be formed from a multi-layer material including an absorbent lining layer, a heat reflective layer, and a foil outer layer. The absorbent lining is configured to contact the patient in use, and is aptly a relatively soft lint or fibrous material. The heat reflective layer may be disposed between the absorbent lining layer and the foil outer layer. The foil outermost layer is aptly waterproof or water repellent. The three layers together provide a thermally insulating and waterproof outer sleeve to help to keep the patient warm and dry and maintain a constant body temperature. The outer layer may additionally be configured to be of high visibility to help maintain patient visibility in lower light conditions. Aptly, the outer sleeve 106 is formed from a medically approved material. One example of a suitable material is Mediwrap^{®} material, which is often used as a passive patient warming system (e.g. a blanket). Mediwrap^{®}, available from Cantel Medical UK, is highly thermally retentive, for single patient use, includes a soft and absorbent inner layer, has a draft and waterproof outer shell, allows easy access to surgical field, and is a cost effective alternative to active warming.

The outer surface of the outer sleeve 106 may be printed with indicia, for example instructions for use and/or branding.

The expansion chamber 104 may aptly be fixed to the outer sleeve 106. For example, the expansion chamber may be stitched onto the outer sleeve to form a closed splint sleeve or container. In other examples, the expansion chamber may be adhered to the outer sleeve using a suitable glue, for example. In other examples, the expansion chamber may be attached to the outer sleeve via a single attachment point, for example a glue line.

The expansion chamber is configured to contain the expanded foam to help prevent the foam from adhering to the outer sleeve 106. As such, flexibility of the outer sleeve can be maintained to help form the splinting apparatus around a body part.

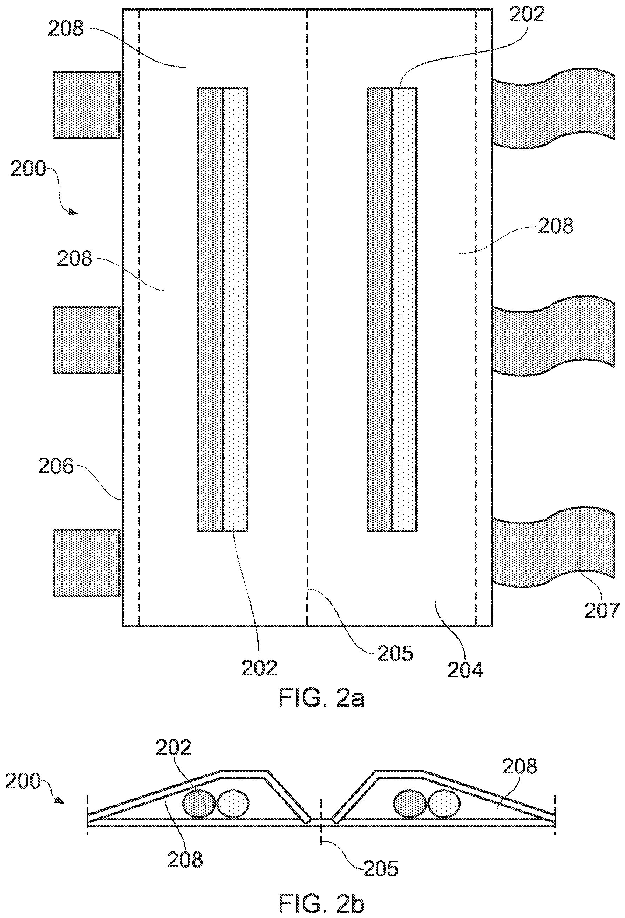

Figures 2a and 2b illustrate another example of a splinting apparatus 200. The splinting apparatus 200 is similar to that shown in Figure 1, but includes a plurality of retaining pouches 202. In this example the splitting apparatus includes two retaining pouches 202. Each retaining pouch 202 is substantially the same as the retaining pouch 102 described in relation to Figure 1, so for brevity will not be described again in detail.

In this example, each of the retaining pouches 202 are enclosed within a single expansion chamber 204. The expansion chamber 204 may be configured similarly to the expansion chamber 104 of the example shown in Figure 1. However, in this example, the expansion chamber 204 includes two separate guiding portions 208 each enclosing a single respective retaining pouch 202. In this example, each of the guiding portions 208 are separated by stitch lines 205. Alternative means for separating guiding portions may include heat welded seals.

Each of the retaining pouches 202 may be activated in the same way as described above in relation to Figure 1 by applying a force to break the sacrificial seam to thereby mix the first and second precursors. The expanding foam from each retaining pouch 202 is enclosed and guided in its respective guiding portion 208 such that two substantially elongate and separate foam supports are formed within each guiding portion of the expansion chamber 204.

Similarly to the example of Figure 1, the splinting apparatus 200 may be placed around the body part of a patient whilst the foam is expanding so that the foam conforms to the body part. The example shown in Figures 2a and 2b may be particularly suitable for splinting of a limb (e.g. an arm or leg).

This example also includes an outer sleeve 206 enclosing the expansion chamber 204. The outer sleeve 206 includes securing elements 207. In this example, the outer sleeve 206 includes three spaced apart securing elements 207. In this example, each of the securing elements 207 include elastane fasteners having a unidirectional stretch. The securing elements are coupled to the outer sleeve 206 such that they extend transverse to the longitudinal direction of the outer sleeve 206. In this way, the unidirectional stretch of the fasteners is also transverse to the longitudinal direction of the outer sleeve 206 such that the fasteners provide an elastic coupling of the splinting apparatus to the patient, which may provide for improved comfort. The securing elements may be secured in place on the opposite side of the outer sleeve by a hook and loop fastening.

The outer sleeve may be configured similarly to the outer sleeve 106 described in relation to Figure 1, including an envelope for retaining the expansion chamber 204. The expansion chamber 204 may be secured to an inner surface of the outer sleeve 206 to maintain the expansion chamber 204 in a desired position relative to the outer sleeve 206.

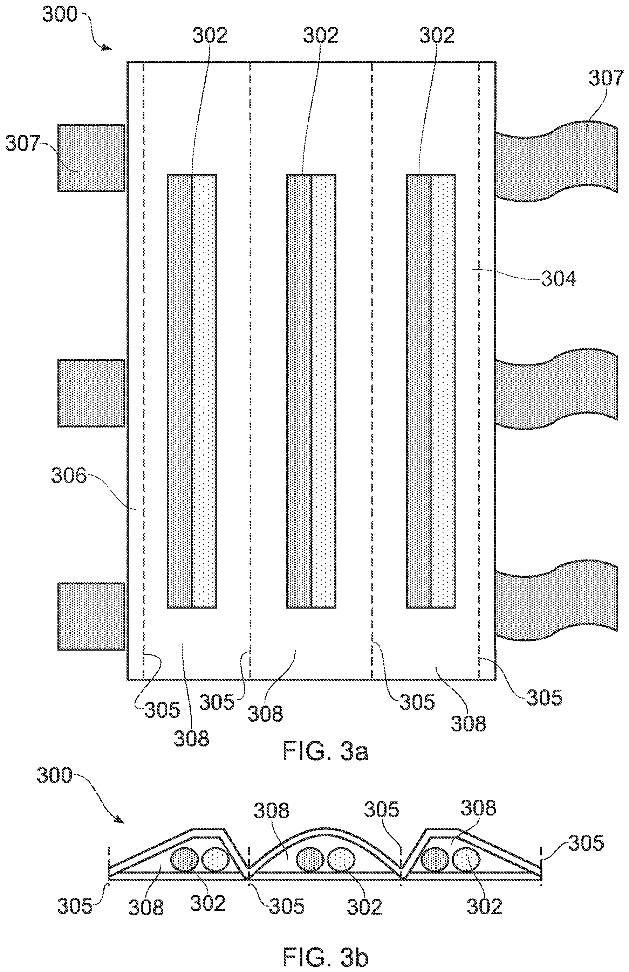

Figures 3a and 3b illustrate another example of a splinting apparatus 300. The splinting apparatus 300 is similar to that shown in Figures 2a , but includes a three retaining pouches 302. Each retaining pouch 302 is substantially the same as the retaining pouch 302 described in relation to Figure 1, so for brevity will not be described again in detail.

In this example, each of the retaining pouches 302 are enclosed within a single expansion chamber 304. The expansion chamber 304 may be configured similarly to the expansion chamber 104 of the example shown in Figure 1. However, in this example, the expansion chamber 304 includes three separate guiding portions 208 each enclosing a single respective retaining pouch 302. In this example, each of the guiding portions 308 are separated by stitch lines 305. Alternative means for separating guiding portions may include heat welded seals.

Each of the retaining pouches 302 may be activated in the same way as described above in relation to Figure 1 by applying a force to break the sacrificial seam to thereby mix the first and second precursors. The expanding foam from each retaining pouch 302 is enclosed and guided in its respective guiding portion 308 such that three substantially elongate and separate foam supports are formed within each guiding portion of the expansion chamber 304.

Similarly to the example of Figure 1, the splinting apparatus 300 may be placed around the body part of a patient whilst the foam is expanding so that the foam conforms to the body part. The example shown in Figures 3a and 3b may also be particularly suitable for splinting of a limb (e.g. an arm or leg).

This example also includes an outer sleeve 306 enclosing the expansion chamber 304. The outer sleeve 306 includes securing elements 307. The securing elements 307 may be configured similarly to the securing elements 207 described in relation to Figures 2a and 2b, so for brevity will not be described again in detail.

The outer sleeve 306 may be configured similarly to the outer sleeve 106 described in relation to Figure 1, including an envelope for retaining the expansion chamber 304. The expansion chamber 304 may be secured to an inner surface of the outer sleeve 306 to maintain the expansion chamber 304 in a desired position relative to the outer sleeve 306.

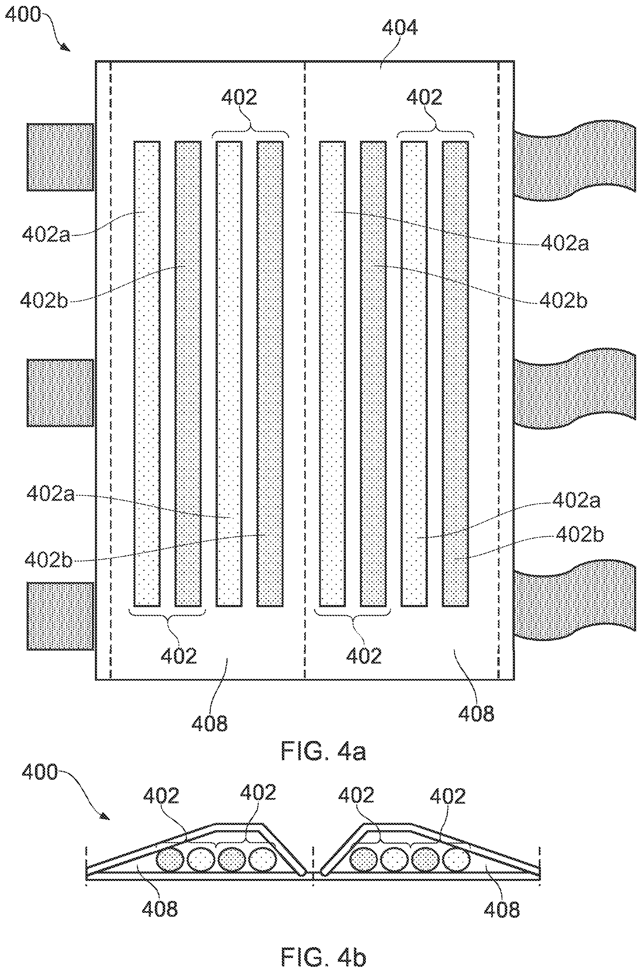

Figures 4a and 4b illustrate a further example of a splinting apparatus 400. In this example, the splinting apparatus is configured very similarly to the splinting apparatus 200 shown in Figures 2a and 2b with the exception that the splinting apparatus 400 includes four retaining pouches 402. In this example, the expansion chamber 404 is divided into two guiding portions 408. Each guiding portion 408 contains two retaining pouches 402.

The retaining pouches 402 in this example each include first and second separate portions 402a, 402b. Each of the first and second separate portions 402a, 402b includes a respective sacrificial seam (not shown). The sacrificial seam may be a weakened point in each of the first and second portions, which may break upon application of a force to the retaining pouch. Thus, the retaining pouches 402 may be activated similarly to the other retaining pouches described herein by applying a force to the retaining pouch to break the sacrificial seam of each of the first and second portions to thereby allow mixing of the first and second precursors.

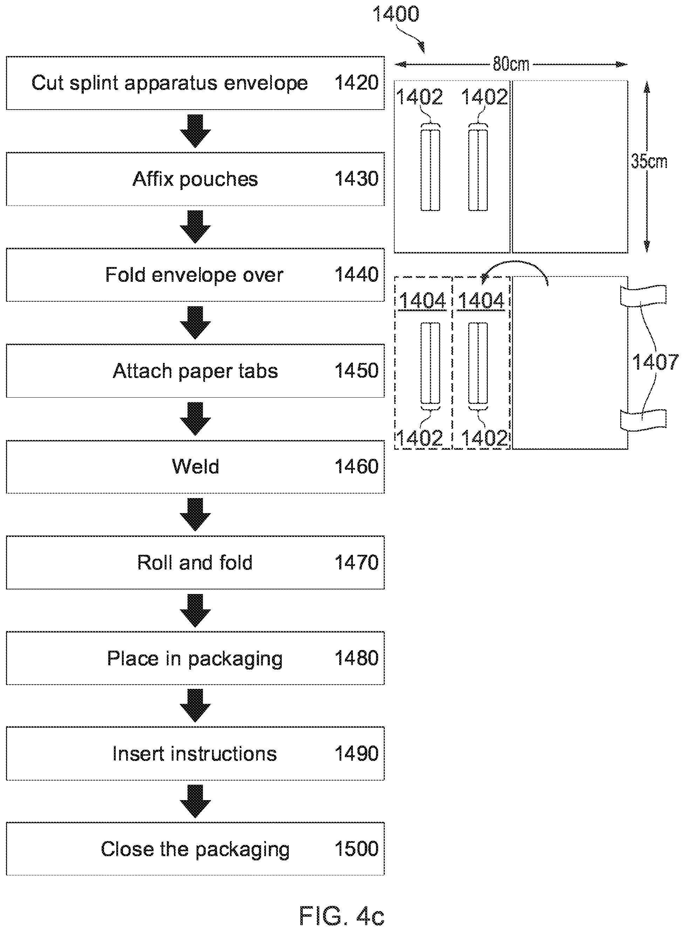

Figure 4c illustrates an exemplary method of assembling and packaging a splinting apparatus 1400. The splinting apparatus 1400 includes two retaining pouches 1402. Each retaining pouch includes a first portion 1402a and a second portion 1402. The splinting apparatus 1400 and the retaining pouch 1402 is similar to that shown in Figure 2a, so for brevity will not be described again in detail. An expansion chamber 1404 encloses the retaining pouch 1404. The expansion chamber 1404 contains the expanding foam and helps to guide the expanding foam into the desired areas. In this example, the expansion chamber 1404 is substantially rectangular in shape and may be particularly suited to splinting of a limb. The expansion chamber 1404 may be any other suitable shape depending on the intended use for the splinting apparatus. In this example, the expansion chamber 1404 is enclosed within an outer sleeve. The outer sleeve comprises an envelope for retaining the expansion chamber 1404.

In the depicted example, the envelope of the outer sleeve has a length of 80cm and a width of 35cm. The envelope is cut at 1420 to form two portions of the outer sleeve of equal size, each having a length of 40cm and a width of 35cm. The retaining pouches 1402 are affixed to a first portion of the envelope (at 1430) in this example by stitching, but the retaining pouches may also be placed onto the envelope, or they may be affixed to the envelope by other methods such as via an adhesive. The envelope portion not containing the retaining pouches 1402 is then folded over the envelope portion containing the retaining pouches 1402 at step 1440. Securing elements 1407 are attached to the envelope portion not containing the retaining pouches 1402 (step 1450) and the envelope is welded, at step 1460,on four sides and further welding on a line separating the pairs of retaining pouches 1402. The envelope is rolled and/or folded (step 1470) before it is placed into packaging (1480) along with instructions (step 1490) on how to use the splinting apparatus 1400. The package is then closed (step 1500). In this example, the package is sealed with a tamper-proof seal for safety and security purposes. It should be appreciated that certain steps in this method can be carried out in a difference sequence, some before others. For example, the retaining pouches 1402 may be affixed to the envelope portion at any point before the envelope is folded, and the securing elements may be attached at any point before the envelope is placed into packaging.

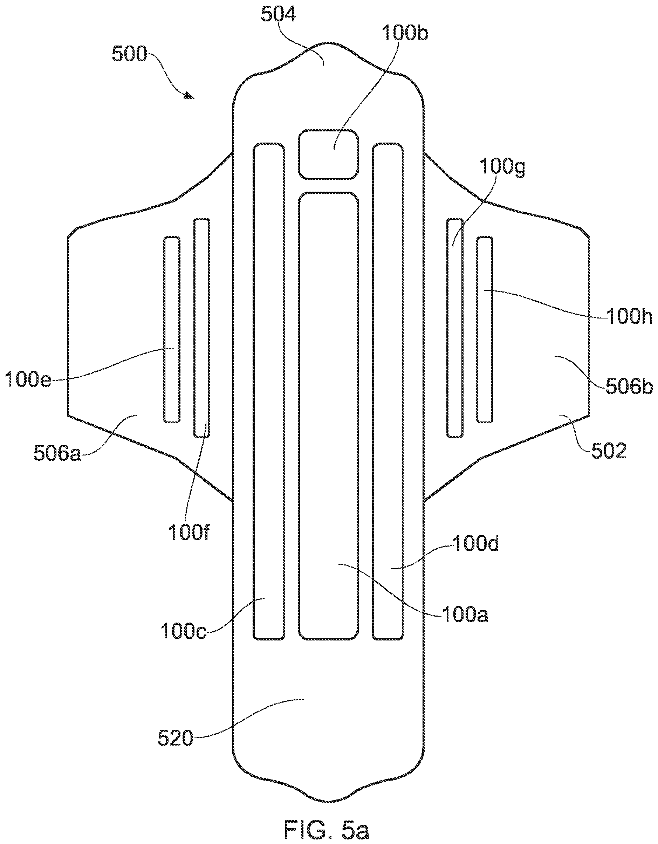

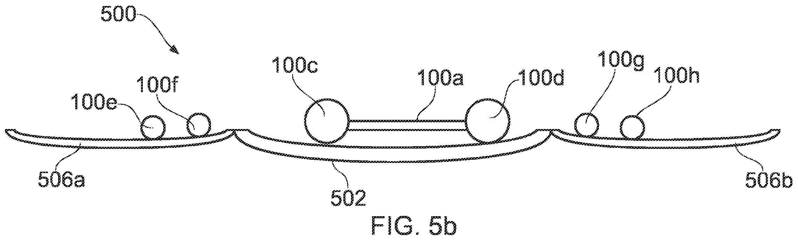

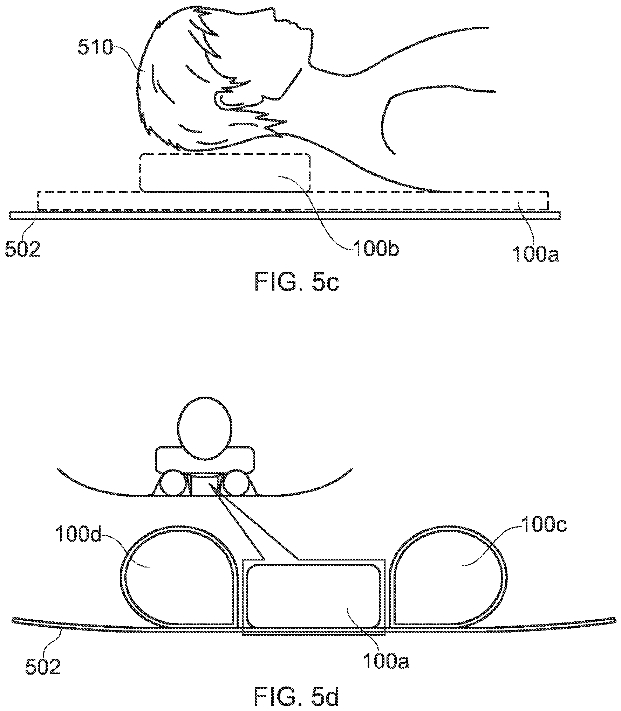

Figures 5a to 5d illustrate an example of a splinting apparatus configured as a stretcher 500. The stretcher includes a plurality of supports 100a-h, and may also be referred to as a splinting system. Each of the supports 100a-h may be configured as a splinting apparatus similar to that described in relation to Figure 1 or may alternatively be configured as a foam pad for providing a soft cushioning to the respective body part of a patient.

In this example, the stretcher 500 is sized and shaped to accommodate the full body of a person 510. The stretcher 500 includes an outer shell 502. The outer shell 502 forms the base of the stretcher 500 and supports the plurality of supports 100a-h. In this example, the supports 100a-h are each secured (e.g. by stitching) onto the outer shell 502. The outer shell 502 in this example includes a central portion 504 for supporting the main body of the person 510 and first and second wing portions 506a, 506b disposed either side of the central portion 504 and configured for wrapping over the body of the person 510 positioned on the central portion 504.

The stretcher 500 includes a main central support 100a positioned on the central portion 504 and configured (sized and shaped) to support the main body of the person 510. The main central support 100a aptly comprises as a relatively soft foam pad configured to provide a cushioning support to the spinal area of the patient. In other examples, the main central support may comprise a splinting apparatus similar to the example of Figure 1 or any of the other examples described herein. The main central support 100a may aptly be configured to be around 450 to 550 mm in thickness, or more aptly 500 mm in thickness.

A head block 100b is located on the central portion 504 over or adjacent to the main central support 100a and is configured to support the head of the person 510. The head block 100b may include a splinting apparatus including a retaining pouch and expansion chamber (similar to that described in relation to Figure 1 or any other example described herein). Alternatively, the head block 100b may include a foam pad (e.g. a preformed foam pad). The foam pad may aptly be formed from a relatively soft foam to provide cushioning support to the head and neck of the patient. The foam pad may be secured onto the main central support 100a or may be secured directly to the outer shell 502 of the stretcher 500 e.g. via stitching or adhesive.

Peripheral supports 100c, 100d are positioned on the central portion 504 either side of the main central support 100a for providing support to peripheral body portions of the person 510. The peripheral supports 100c, 100d aptly each comprise a splinting apparatus similar to that described in Figure 1 or any other examples described herein.

The peripheral supports 100c, 100d may each be configured as elongate tubes extending longitudinally adjacent to the main central support 100a. Each of the elongate tubes may aptly be configured to expand to a diameter of around 750 to 850 mm or more aptly 800 mm. It will be appreciated that the expanded diameter of the elongate tubes may be controlled according to the volume of precursor contained within each of the portions of the retaining pouch, and/or the number of retaining pouches in each tube.

A further two supports 100e-h are provided on each wing portion 506a, 506b, and are configured to conform to the front of the person 510 when the wing portions 506a, 506b are wrapped over the person 510. The further supports 100e-h aptly each comprise a splinting apparatus similar to that described in Figure 1 or any other examples described herein. Similarly to the peripheral supports 100c, 100d, the further supports may each be configured as elongate tubes extending longitudinally along the respective wing portions 506a, 506b. The inner of the elongate tubes (i.e. those closest to the central portion 504) may aptly be configured to expand to a diameter of around 350 to 450 mm or more aptly 400 mm. The outer of the elongate tubes (i.e. those furthest from the central portion 504) may aptly be configured to expand to a diameter smaller than that of the inner elongate tubes. For example, the outer elongate tubes may be configured to expand to a diameter of around 250 to 350 mm or more aptly 300 mm. It will be appreciated that the expanded diameter of the elongate tubes may be controlled according to the volume of precursor supplied to each of the portions of the retaining pouch, and/or the number of retaining pouches in each tube.

Each support 100a-h may be configured similarly to any of the splinting apparatus described herein, or as described above, may be configured as a preformed foam pad. Each support is each sized and shaped to fit the relative portion of the stretcher 500. Where the support 100a-h comprises a splinting apparatus, the splinting apparatus may include an outer sleeve and be secured to the outer shell 502 via the outer sleeve. For example, the outer sleeve may be stitched onto the outer shell 502. In other examples, the outer shell 502 may form an outer sleeve and include a plurality of envelopes for retaining a respective expansion chamber of each of the splinting apparatus 100a-h, mitigating the need for a separate outer sleeve for each splinting apparatus 100a-h. The envelopes of the outer shell 502 may be separated from each other via stitch lines or weld lines, or intermediate adjoining fabric for example.

Where the support 100a-h comprises a preformed foam pad, the foam pad may be secured to the outer shell, e.g. by stitching or adhesive. In other examples, the outer shell may include one or more envelopes configured to retain the foam pad(s) in position.

The outer shell 502 of the stretcher 500 may include an integral carrying bag 520, which may be located at the foot end of the stretcher. The carry bag 520 may be configured to enclose the stretcher in a folded or rolled configuration prior to use. The carry bag 520 may be configured to protect the contents during transportation. In particular, the carry bag 520 may be of sufficient gauge or rigidity to help prevent accidental activation of the supports 100a-h that comprise splinting apparatus by breakage of the sacrificial seams.

In some examples, the stretcher 500 may also include a hood portion (not shown). The hood portion may be located at the head end of the stretcher and may be configured for placing and securing around the head of a patient 510 when in position on the stretcher. The hood portion may aptly be integral with the outer shell and may be formed from the same material as the outer shell. The hood portion is aptly of insulating material to provide passive warming to the patient by retaining head heat. The hood portion may aptly include an elasticated perimeter and/or a draw cord configuration to help secure the hood in position over the patient's head.

When the stretcher 500 is required, a user first removes the stretcher 500 from the integrated carry bag 520 (if present), and then unfolds or unrolls the stretcher 500 into a substantially flat configuration with the supports 100a-h facing upwards. Each of the supports comprising splinting apparatus are then activated by applying a force to the splinting apparatus. In this example, although a torsional (twisting) force may be possible, it can be easier to apply a compressional force to each of the splinting apparatus in order to break the respective sacrifical seams and activate expansion of the foam. Continued agitation of the splinting apparatus after breakage of the sacrifical seams, e.g. by applying a pumping force or sequential compressions to each of the splinting apparatus can help to mix the precursors and provide the activation energy for expansion of the foam.

In this example, the precursors may be selected to provide quicker activation to cure time than the precursors used in the other splinting apparatus described herein. For example, the precursors may be selected such that the activation to cure time is around 1 minute. In some examples, this selection of precursors can give increased off-gas compared to other precursor selections, but in this example this may be beneficial to help with foam expansion. An example of suitable precursors may be those used in Instapak^{®}, manufactured by Sealed Air. In this example, the first precursor may be a Aromatic Isocyanate, for example Polymethylene Polyphenylisocyanate, and the second precursor is a Polyurethane foam resin.

Mixing of the first and second precursors by continued agitation activates expansion of the foam for each splinting apparatus. Whilst the foam is expanding, the patient 510 is positioned in place on the stretcher 500, with their back placed in the central portion 504 resting on the main central support 100a (and possibly also the peripheral supports 100c, 100d). The head of the patient 510 is positioned on the head block 100b 100b. The wing portions 506a, 506b of the stretcher 500 may then be wrapped over the front of the patient 510 and secured in place (e.g. with securing elements, for example including elasticated straps having hook and loop fasteners). The foam of each of the supports 100a-h that include splinting apparatus continues to expand to the final volume and during the expansion conforms to the shape of the patient. After the expanded foam of each of the splinting apparatus has cured (around 1 to 9 minutes after activation, or more aptly around 1 to 3 minutes after activation, or in some examples around 1 minute after activation), the splinting apparatus acts to immobilise the patient 510 in the stretcher 500.

The stretcher 500 may further include grab handles (not shown) to aid lifting of the stretcher.

In some examples, the stretcher may further include an adjustable length lower region (i.e. foot region) for adjusting the stretcher to the length of the patient. The adjustable length lower region may include an adjustable buckle fastener for releasing additional length of the stretcher or for drawing in excess length of the stretcher to the desired length requirements.

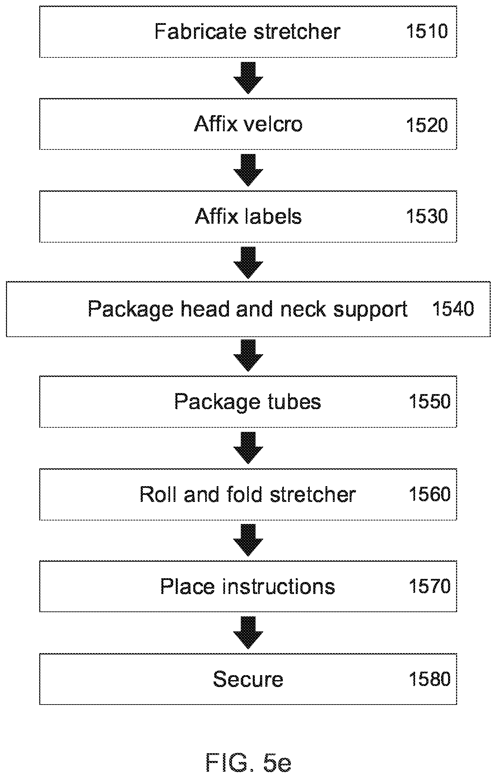

Figure 5e illustrates an example of packaging the stretcher 500 of Figures 5a to 5d. After the stretcher 500 has been fabricated (step 1510), fasteners (step1520) are applied around the supports 100a-h. For example, "hook-and-loop" fasteners are used to secure the supports 100a-h in a spaced apart manner such that three "hook-and-loop" fasteners are used to secure each support 100a-h.A different number of fasteners and fastener types can be used depending on the size and length of the support 100a-h. Labels (step 1530) are affixed to the stretcher 500. For example, safety labels are affixed to the inside and logo labels are affixed to the outside. The stretcher is packaged, for example inside the integrated carry bag 520. The head block 100b is placed inside the carry bag 520, in step 1540, as are the supports (steps 1550). The stretcher 500 is rolled and folded (step 1560) and placed within the carry bag 520. The carry bag 520 may be configured to enclose the stretcher in a folded or rolled configuration The carry bag 520 may be configured to protect the contents during transportation. In this example the carry bag 520 has a handle (not shown) for the user to more easily carry the contents. A instruction card (step 1570) is placed around the handle. The carry bag 520 is placed into a box to be secured (step 1580) and for transporting the stretcher 500.

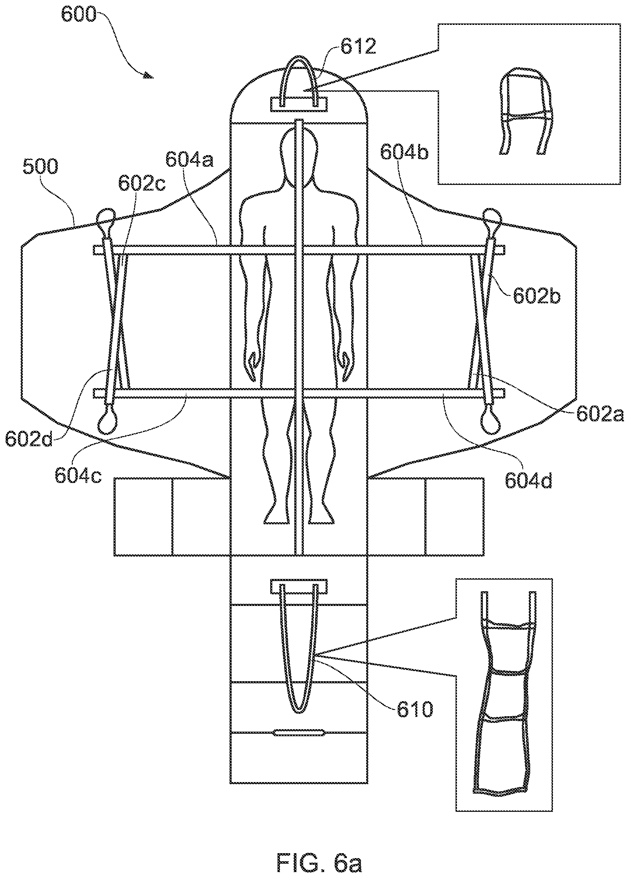

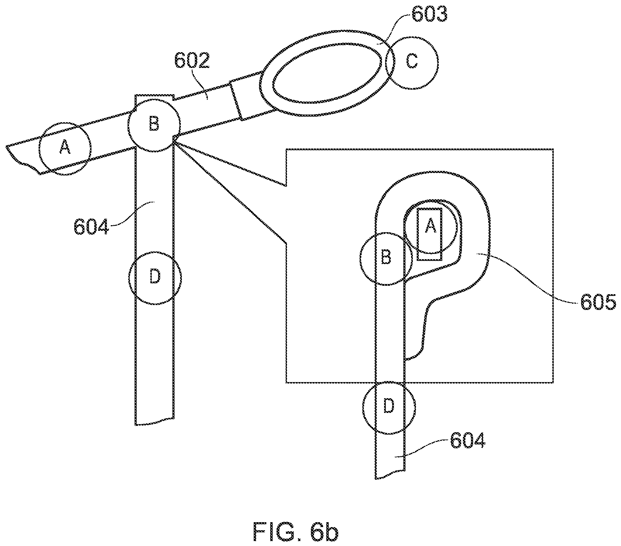

Figures 6a to 6f illustrate a lifting harness arrangement 600 for a stretcher such as that described in relation to Figures 5a to 5d. The harness 600 includes four lifting tapes 602a-d, with two of the lifting tapes located on each side of the harness. Four transverse tapes 604a-d extend transversly across the strectcher 500 and intersect with a corresponding lifting tape 602a-d. The transverse tapes 604a-d may each be coupled to the respective lifting tape 602a-d at the intersection (e.g. by stitching). The free end of each of the transverse tapes 604a-d includes a loop 605, which may be a stiched loop, through which an end of a corresponding lifting tape 602a-d may pass (see Figure 6b).

In one example each of the lifting tapes 602a-d include a loop 603 at one end, which may be a preformed flat loop carrying handle attached to the lifting tape (as shown in Figure 6b) or in another example may be formed by stitching back a length of the lifting tape 602a-d to form a loop.

The harness may also include a strengthening tape 606 running along substantially the length of the stretcher 500.

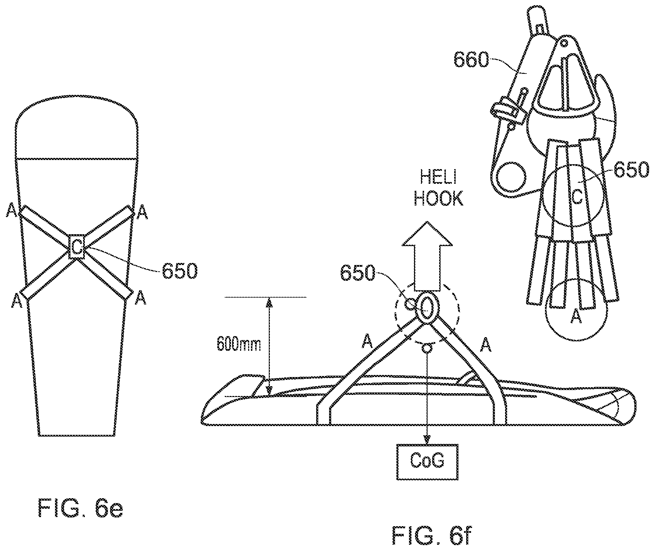

As shown in Figures 6e and 6f, the loops 603 of each lifting tape may be gathered together to a single point for attachment to a hook 660, for example a heli-hook or other lifting hook.

The harness is aptly coupled to the the outer or underside of the stretcher, i.e. the opposite side to the support elements of the stretcher 500 in Figures 5a to 5d. The harness may aptly the coupled to the stretcher 500 by stitching.

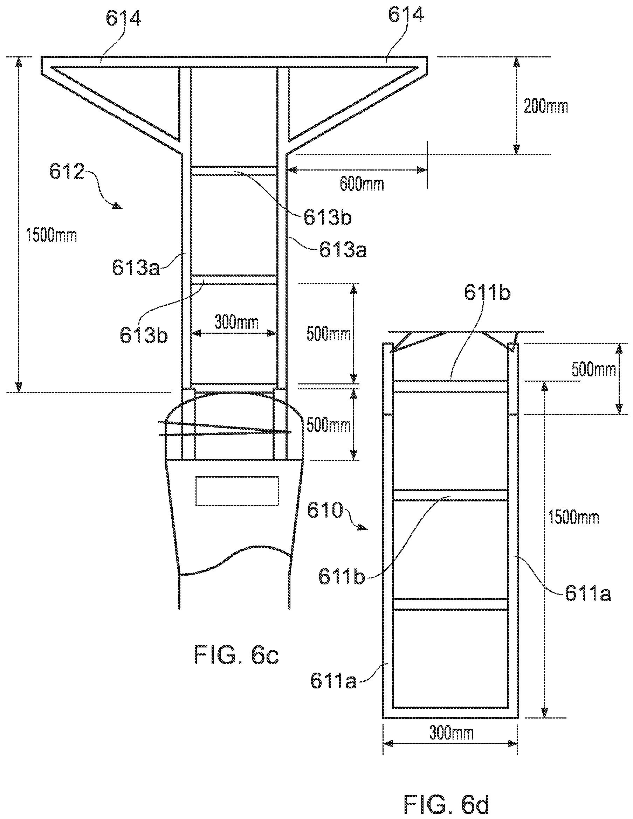

The harness may also include a foot tow handle 610. The foot tow handle 610 may be coupled to the lower (foot) end of the stretcher 500, e.g. via stitching. The foot tow handle 610 may be fabricated from a flat web sling and conform to BS EN1492-1. The webbing may be about 90 mm in width and around 450 g/m. The foot tow handle 610 may be configured as a ladder shape including longitudinal side straps 611a and transverse straps 611b coupled between the longitudinal side straps 611a (see Figure 6d).

The harness may additionally include a head tow handle 612. The head tow handle 612 may be coupled to the upper (head) end of the stretcher 500, e.g. via stitching. The head tow handle 612 may be fabricated from a flat web sling and conform to BS EN1492-1. The webbing may be about 90 mm in width and around 450 g/m. The head tow handle 612 may be configured as a ladder shape including longitudinal side straps 613a and transverse straps 613b coupled between the longitudinal side straps 613a (see Figure 6c). Further lateral extensions 614 may be provided at the end of the head tow handle 612 distal from the coupling to the stretcher 500.

The end of the head and/or foot tow handles may include reinforced strap regions for coupling to the stretcher 500.

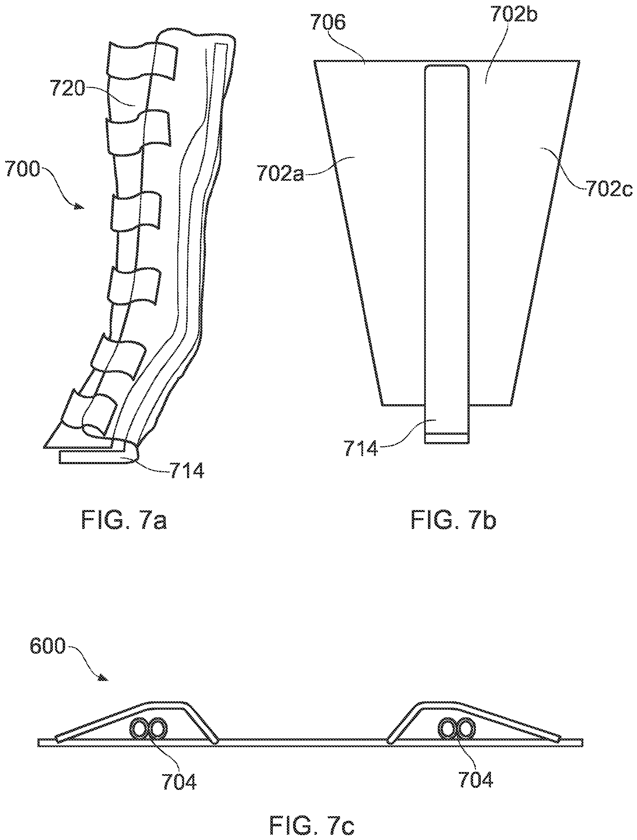

Figures 7a to 7c illustrate a further example of a splinting apparatus 700. The splinting apparatus 700 is similar to the splinting apparatus 200, 300 shown in Figures 2 and 3, but is configured for veterinary long leg splinting, e.g. the leg 720 of canine, equine or cattle. The splinting apparatus 700 in this example includes an outer sleeve 706 having three envelopes 702a-c arranged in parallel and extending longitudinally along the outer sleeve 706. First and second envelopes 702a, 702c disposed on first and second sides of the outer sleeve 706 are configured to each enclose an expansion chamber containing a respective one or more retaining pouch 704 (see Figure 7c). In this example, each outer envelope 702a, 702c encloses an expansion chamber containing two retaining pouches 704. In other examples, any number of retaining pouches, or expansion chambers or outer sleeve envelopes may be present according to the shape and size of the leg to be splinted.

A further central envelope 702b is disposed between each outer envelope 702a, 702c and is configured to enclose a metal splint 714. The metal splint 714 may extend longitudinally along the central envelope 702b and may include a hook portion at the lower end for positioning under the foot or hoof of the animal leg 720. Aptly, the metal splint 714 may be an aluminium splint, though other metals may also be suitable. The metal splint can help to provide additional support for long limb splinting above the support provided by the expanded foam contained in each of the expansion chambers.

The splinting apparatus 700 may be operated in substantially the same way as the splinting apparatus 100 of Figure 1 by applying a torsional force to the splinting apparatus to break the sacrificial seam(s) of each of the retaining pouches 704. This begins the mixing of the first and second precursors and the foam starts to expand. Whilst the foam is expanding, the splinting apparatus 700 can be positioned around the animal leg 720 with the hook of the metal splint positioned under the hoof (or foot). The outer sleeve 706 may then be wrapped around the leg and fastened in position. In this example, the splinting apparatus includes securing elements (e.g. elasticated straps including hook and loop fasteners), which may be fastened together to hold the splinting apparatus in position.

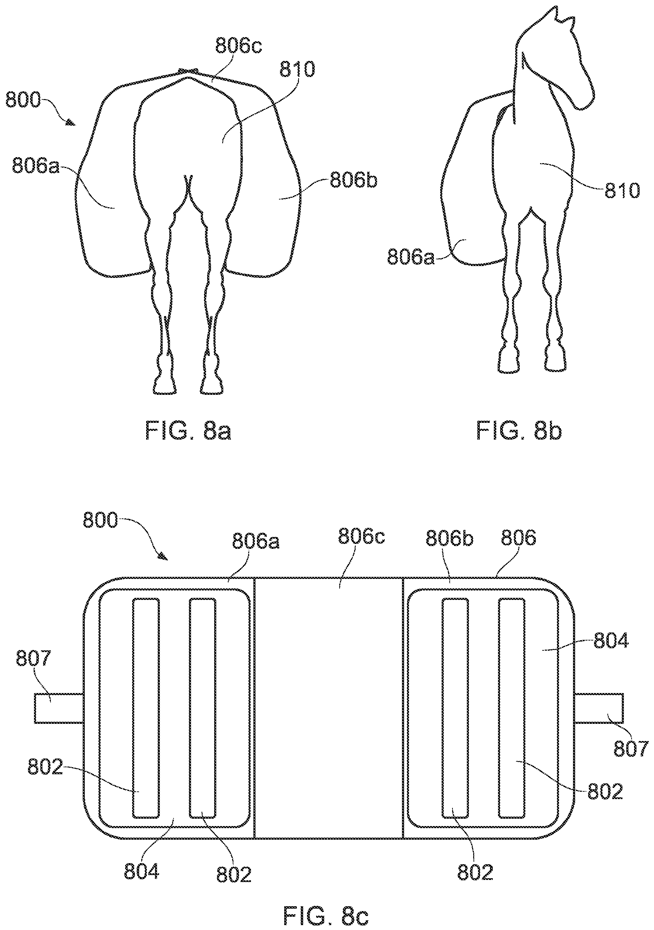

Figures 8a to 8c illustrate a freight blanket 800 including a plurality of splinting apparatus 100. The freight blanket 800 may be used to protect livestock, e.g. horses, during transportation. This is important to prevent injury of the livestock during particularly turbulent journeys, e.g. air or road travel.

Figures 8a and 8b illustrate the freight blanket 800 in situ on a horse 810. Figure 8c illustrates the freight blanket 800 in a flat configuration.

The freight blanket 800 includes an outer sleeve 806 having first and second side envelopes 806a, 806b and a central joining section 806c separating the first and second side envelopes 806a, 806b. The central joining section 806c is configured for positioning over the back of a horse 810 and supporting each of the side envelopes 806a, 806b to hang either side of the horse 810.

Each of the first and second side envelopes 806a, 806b are configured to enclose a respective expansion chamber 804, which may be secured to an inner side the respective envelope 806a, 806b (e.g. via adhesive). The expansion chambers 804 may be configured similarly to any of the other expansion chambers described herein.

Each of the expansion chambers 804 includes first and second retaining pouches 802 (though any other number of retaining pouches may be used according to the desired side of the blanket). Similarly to the examples above, the retaining pouches may be fixed to the inside of the expansion chamber 804 (e.g. with adhesive). The retaining pouches 802 may be configured similarly to the retaining pouch 102 described in relation to Figure 1 including first and second portions, so for brevity will not be described again in detail.

The blanket 800 also includes securing elements 807 (e.g. elasticated straps including hook and loop fasteners) for extending under the belly of the horse and securing the blanket in position.

In use, the horse (or other animal) may be positioned in the transportation stall or container. The blanket may then be activated by applying a torsional (twisting) or a compressional force to the retaining pouches on either side of the blanket 800 to break the sacrificial seam(s) of the retaining pouches. Whilst the foam begins to expand, and before curing, the blanket 800 may then be located over the horse with the central section 806c resting over the horse's back and each of the side envelopes 806a, 806b hanging either side of the horse (as showing in Figures 8a and 8b). The securing elements 807 may then be fastened in place around the horse's belly.

The expanding foam will then conform to the shape of the horse and also any surrounding obstacles (e.g. stall walls) and once cured will secure the horse 810 in position and/or provide protection to the horse to help prevent injury from accidental collision with adjacent obstacles.

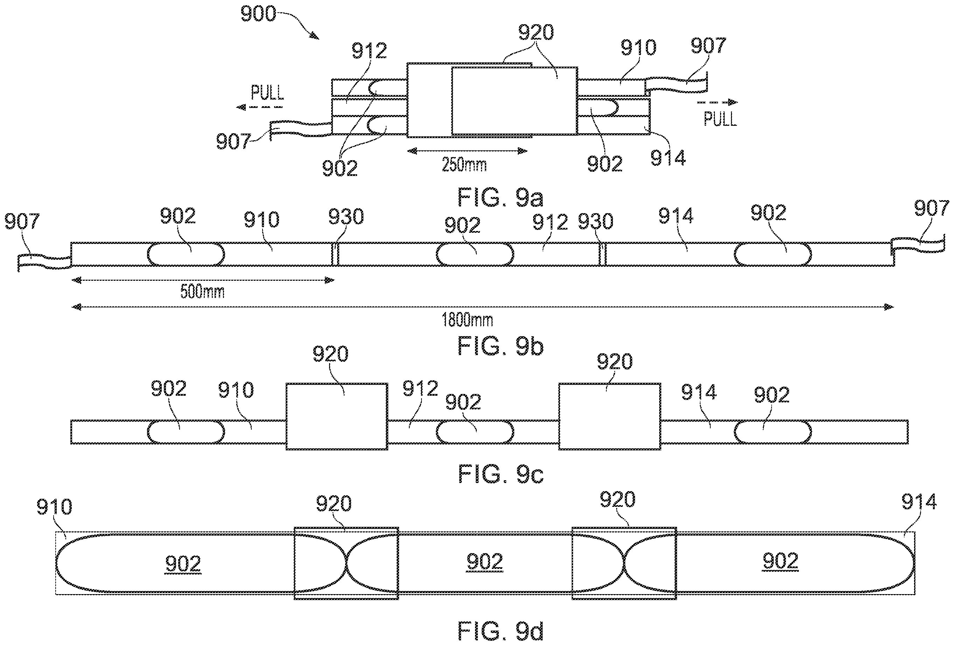

Figures 9a to 9d illustrate an example of a splinting apparatus 900 in a folded state. By folding the apparatus 900, transportation and deployment is made easier. The apparatus 900 includes a single fabric tube forming a first expansion chamber 910, a second expansion chamber 912 and a third expansion chamber 914 as shown in Figure 9a. Each expansion chamber 910, 912, 914 contains a retaining pouch 902. Each retaining pouch 902 and each expansion chamber 910, 912, 914 is similar to the retaining pouch and the expansion chamber in Figure 3a, so for brevity will not be described again in detail. By providing multiple expansion chambers and respective retaining pouches, the support can be made as long as is required and expansion of the foam in each expansion chamber is swift and consistent.

The first expansion chamber 910 has a pull tab 907 disposed on a distal end and the third expansion chamber has a pull tab 907 disposed on a proximal end. The assembly 900 further includes two support collars 920, an inner support collar being telescopically received inside an outer support collar such that the inner support collar may slide relative to the outer support collar. Although the inner support collar is telescopically received inside the outer support collar, the support collars 920 are separable from one another. In this example, the support collars 920 are 250mm long. The inner collar has a diameter of 73mm and the outer collar has a diameter of 75mm. A user pulls on the pull tabs 907 to move the splinting apparatus tube assembly 900 from a folded state to an unfolded state.

The splinting apparatus 900 in an unfolded state is illustrated in Figure 9b. Once unfolded, the expansion chambers 910, 912, 914 are coaxial. In the unfolded state, the interface between the first expansion chamber 910 and the second expansion chamber 912 is welded to create a welded seam 930 therebetween, and the second expansion chamber is separated from the third expansion chamber 914 by another welding seam 930. The collar supports 920 are not shown in Figure 9b to show the welding seams 930.

Figure 9c illustrates the collar supports 920 aligned with the welding seams 930. When the assembly 9c is unfolded, the collar supports 920 also move such that the collar supports 920 separate from one another. The collar supports 920 align with the welding seams 930 and cover them. It this way the expanded splinting apparatus tube 900 has a substantially uniform diameter and the collar supports 920 provide further protection to the welded seams 920.

Each of the retaining pouches 902 may be activated in the same way as described in relation to Figure 1 by applying a force to break the sacrificial seam to thereby mix the first and second precursors. Figure 9d illustrates the splinting apparatus tube assembly 900 where the retaining pouches 902 are activated. The expansion of the retaining pouches 902 expands the expansion chambers 902 in the tube. This expansion is supported by the support collars 920.

Various modifications to the specific examples above may be possible. For example, the sacrificial seam by be formed by any appropriate means. For example, the sacrificial seam may be formed by a heat weld of opposing sides of the retaining pouch. In other examples, the sacrificial seam may be formed by a weakening line in the join between the first and second portions of the retaining pouch, or a perforation. In another example, the sacrificial seam may be formed by, a tear strip or a pull cord that may be pulled by a user to activate the splinting apparatus. Aptly, the sacrificial seam may be configured such that a minimum force is required to break the seam. For example, the minimum force required to break the seam may be to compress a sachet or pouch of polymer until failure of the packaging occurs. The peak compressive force may be around 300 to 400 N. To test the peak compressive force, the test was deemed complete when a sudden drop in load was detected or release of the sample was evident. Test results were in the region of 300 - 400N.

Alternative first and second precursors to those described above may be suitable for forming the expanded polymer foam. Suitable precursors will be known to those skilled in the art, for example various monomer precursors. The precursors may be selected according to desired properties of the expanded foam (e.g. expansion volume, cure time, exothermic reaction temperature).

In some examples, it may be advantageous to select the precursors such that the resultant polymer foam is substantially buoyant. This can be particularly advantageous for water rescue operations.

The expansion chambers described herein may alternatively be configured as a single sheet attached to the surrounding outer sleeve to form an enclosed chamber. The sheet may be a polymer sheet and may be stitched or heat welded around the periphery to form an enclosed expansion chamber in which to guide and contain the expanding foam. In some examples, the sheet may be attached to the outer sleeve at intermediate locations to divide the expansion chamber into two or more guiding portions.

In some examples, the expansion chamber may aptly be waterproof to help contain the expanding foam.

The outer sleeves described herein may be formed of any flexible material suitable for containing the expansion chamber. Other example materials may include Tyvek ^{®} (DuPont), ripstop nylons or treated paper.

Any of the outer sleeves described herein may include an integral carry bag configured to contain the splinting apparatus or system during transportation.

The outer sleeve may be configured to have a plurality of envelopes each for enclosing a respective one or more expansion chamber. In other examples the outer sleeve may have a single envelope for enclosing one or more expansion chamber.

Any of the securing elements described herein may include a securing strap having a fastener. The securing strap may be coupled to the outer sleeve of the apparatus (or the outer shell) and may be configured to have unidirectional stretch. The unidirectional stretch may help to give increased flexibility in the apparatus to provide improved comfort to the patient or user. The fastener may comprise a hook and loop fastener to secure the strap in a closed position. Alternative fasteners may comprise buckles, or ties for example.

Different forces may be possible to break the sacrificial seam and activate the splinting apparatus. Aptly the sacrificial seam is broken by a torsional force. This can help to promote even blending of the precursors and introduces an activation energy sufficient for expansion of the polymer foam. Alternatively, a compression force may be applied to the splinting apparatus to force breakage of the sacrificial seam. This method may need an agitation, for example additional pumping action or sequential compressions after breakage to help promote mixing of the precursor and provide activation energy for activating expansion of the polymer foam.

Although the examples described above are configured for particular applications, the splinting apparatus may be configured for many different applications. For example, the splinting apparatus may be configured for any of pelvic, neonate, half limb, full limb, full body, wrist, ankle, hand, head and neck splinting. The splinting apparatus may also be configured for veterinary splinting, including freight blankets, and animal legs splints (e.g. for cattle, equine, canine, and other large mammals).

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any any embodiments which fall within the scope of the claims.

## Claims

1. A splinting apparatus (100, 200, 300, 400, 700) comprising:
a retaining pouch (102, 202, 302, 402, 704, 802) having a first portion (102a, 402a) containing a first precursor, a second portion (102b, 402b) containing a second precursor, and at least one sacrificial seam (103) fluidly separating the first portion (102a, 402a) from the second portion (102b, 402b); and
an expansion chamber (104, 204, 304, 804) enclosing the retaining pouch (102, 202, 302, 402, 704, 802);
wherein the at least one sacrificial seam (103) is breakable upon application of a force to the retaining pouch (102, 202, 302, 402, 704, 802) to thereby fluidly connect the first and second portions (102a, 102b, 402a, 402b) of the retaining pouch (102, 202, 302, 402, 704, 802) such that the first and second precursors mix together, and
wherein mixing of the first and second precursors forms a curable expanded polymer foam, and
**characterised in that** the expansion chamber (104, 204, 304, 804) has a volume that is about 110% of the volume of the expanded polymer foam.

2. A splinting apparatus according to claim 1, wherein:
the first portion (102a, 402a) and the second portion (102b, 402b) of the retaining pouch (102, 202, 302, 402, 704, 802) are coupled together by the sacrificial seam (103); or
each of the first portion (102a, 402a) and the second portion (102b, 402b) comprise one of the at least one sacrificial seam (103).

3. A splinting apparatus according to any preceding claim, wherein:
the first and second precursors are miscible; and/or
each of the first and second precursors are a monomer precursor.

4. A splinting apparatus according to any preceding claim, wherein mixing of the first and second precursors provides an exothermic reaction providing heat having a temperature from 20 degrees Celsius to 37 degrees Celsius.

5. A splinting apparatus according to any preceding claim, wherein the expanded polymer foam:
is configured to cure within 1 to 9 minutes of breaking of the sacrificial seam; and/or
is a closed-cell foam; and/or
is mouldable prior to curing.

6. A splinting apparatus according to any preceding claim, wherein:
the volume of the expanded polymer foam is about 20 times the total volume of the precursors prior to mixing; and/or
the retaining pouch (102, 202, 302, 402, 704, 802) is configured to burst upon expansion of the first and second precursors as they mix to form the expanded polymer foam.

7. A splinting apparatus according to any preceding claim, wherein:
each of the first and second portions (102a, 102b, 402a, 402b) of the retaining pouch (102, 202, 302, 402, 704, 802) are configured as elongate tubes; and/or
the first and second portions (102a, 102b, 402a, 402b) extend along from 50% to 90% of the length of the expansion chamber (104, 204, 304, 804).

8. A splinting apparatus according to any preceding claim, further comprising at least one further retaining pouch (202).

9. A splinting apparatus according to any preceding claim, wherein the expansion chamber (104, 204, 304, 804) is formed as a bladder or a bag.

10. A splinting apparatus according to any preceding claim, further comprising an outer sleeve (106, 206, 306, 706, 806) enclosing the expansion chamber (104, 204, 304, 804), and optionally wherein the expansion chamber (104, 204, 304, 804) is secured to an internal surface of the outer sleeve (106, 206, 306, 706, 806).

11. A splinting apparatus according to claim 10, wherein the outer sleeve (106, 206, 306, 706, 806) comprises an envelope (702a, 702b, 702c, 806a, 806b) configured to retain the expansion chamber (104, 204, 304, 804), and optionally wherein the envelope (702a, 702b, 702c, 806a, 806b) comprises an upper layer and a lower layer, and wherein the upper layer and the lower layer are secured together to form the envelope.

12. A splinting apparatus according to claim 10 or claim 11, wherein the outer sleeve (106, 206, 306, 706, 806):
is formed from a flexible material; and/or
further comprises securing elements (207, 307) for securing the splinting apparatus around a body part of a patient; and/or
is sized and shaped to form a C-shaped splint; and/or
is configured to further enclose a metal splint (714) for splinting a relatively long limb; and/or
comprises a joining portion (806c) for separating first and second envelopes (806a, 806b), each of the first and second envelopes (806a, 806b) retaining an expansion chamber (804).

13. A splinting system comprising a plurality of splinting apparatus according to any preceding claim.

14. A method of activating the splinting apparatus of any of claims 1 to 12, the method comprising applying a force to the splinting apparatus (100, 200, 300, 400, 700) to break the sacrificial seam (103), and optionally wherein the step of applying a force comprises applying a compressional force to the splitting apparatus (100, 200, 300, 400, 700).

15. A method according to claim 14, wherein the step of applying a force comprises twisting the splinting apparatus (100, 200, 300, 400, 700) to provide a torsional force to the splinting apparatus (100, 200, 300, 400, 700), and optionally comprises twisting the splinting apparatus (100, 200, 300, 400, 700) in a clockwise and an anti-clockwise direction.

## Patentansprüche

1. Gerät zur Schienung (100, 200, 300, 400, 700), umfassend:
einen Aufbewahrungsbeutel (102, 202, 302, 402, 704, 802) mit einem ersten Abschnitt (102a, 402a), der einen ersten Vorläufer enthält, einem zweiten Abschnitt (102b, 402b), der einen zweiten Vorläufer enthält, und mindestens einer Opfernaht (103), die den ersten Abschnitt (102a, 402a) fluidisch von dem zweiten Abschnitt (102b, 402b) trennt; und
eine Expansionskammer (104, 204, 304, 804), die den Aufbewahrungsbeutel (102, 202, 302, 402, 704, 802) umschließt;
wobei die mindestens eine Opfernaht (103) bei Anwendung einer Kraft auf den Aufbewahrungsbeutel (102, 202, 302, 402, 704, 802) aufbrechbar ist, um dadurch den ersten und zweiten Abschnitt (102a, 102b, 402a, 402b) des Aufbewahrungsbeutels (102, 202, 302, 402, 704, 802) derart fluidisch zu verbinden, dass sich der erste und zweite Vorläufer miteinander vermischen, und
wobei durch das Vermischen des ersten und zweiten Vorläufers ein aushärtbarer expandierter Polymerschaum gebildet wird, und
**dadurch gekennzeichnet, dass** die Expansionskammer (104, 204, 304, 804) ein Volumen aufweist, das etwa 110 % des Volumens des expandierten Polymerschaums beträgt.

2. Gerät zur Schienung nach Anspruch 1, wobei:
der erste Abschnitt (102a, 402a) und der zweite Abschnitt (102b, 402b) des Aufbewahrungsbeutels (102, 202, 302, 402, 704, 802) durch die Opfernaht (103) aneinander gekoppelt sind; oder
jeder von dem ersten Abschnitt (102a, 402a) und dem zweiten Abschnitt (102b, 402b) eine der mindestens einen Opfernaht (103) umfasst.

3. Gerät zur Schienung nach einem der vorhergehenden Ansprüche, wobei:
der erste und zweite Vorläufer mischbar sind; und/oder
jeder von dem ersten und zweiten Vorläufer ein Monomervorläufer ist.

4. Gerät zur Schienung nach einem der vorhergehenden Ansprüche, wobei durch das Vermischen des ersten und zweiten Vorläufers eine exotherme Reaktion bereitgestellt wird, die Wärme mit einer Temperatur von 20 Grad Celsius bis 37 Grad Celsius bereitstellt.

5. Gerät zur Schienung nach einem der vorhergehenden Ansprüche, wobei der expandierte Polymerschaum:
dazu konfiguriert ist, innerhalb von 1 bis 9 Minuten nach dem Aufbrechen der Opfernaht auszuhärten; und/oder
ein geschlossenzelliger Schaumstoff ist; und/oder
vor dem Aushärten formbar ist.

6. Gerät zur Schienung nach einem der vorhergehenden Ansprüche, wobei:
das Volumen des expandierten Polymerschaums etwa das 20-fache des Gesamtvolumens der Vorläufer vor dem Vermischen beträgt; und/oder
der Aufbewahrungsbeutel (102, 202, 302, 402, 704, 802) dazu konfiguriert ist, bei Ausdehnung des ersten und zweiten Vorläufers zu platzen, wenn diese sich vermischen, um den expandierten Polymerschaum zu bilden.

7. Gerät zur Schienung nach einem der vorhergehenden Ansprüche, wobei:
jeder von dem ersten und zweiten Abschnitt (102a, 102b, 402a, 402b) des Aufbewahrungsbeutels (102, 202, 302, 402, 704, 802) als längliche Röhren konfiguriert ist; und/oder
sich der erste und zweite Abschnitt (102a, 102b, 402a, 402b) über 50 % bis 90 % der Länge der Expansionskammer (104, 204, 304, 804) erstrecken.

8. Gerät zur Schienung nach einem der vorhergehenden Ansprüche, ferner umfassend mindestens einen weiteren Aufbewahrungsbeutel (202).

9. Gerät zur Schienung nach einem der vorhergehenden Ansprüche, wobei die Expansionskammer (104, 204, 304, 804) als Blase oder Tasche gebildet ist.

10. Gerät zur Schienung nach einem der vorhergehenden Ansprüche, ferner umfassend eine Außenhülse (106, 206, 306, 706, 806), welche die Expansionskammer (104, 204, 304, 804) umschließt, und wobei optional die Expansionskammer (104, 204, 304, 804) an einer Innenfläche der Außenhülse (106, 206, 306, 706, 806) befestigt ist.

11. Gerät zur Schienung nach Anspruch 10, wobei die Außenhülse (106, 206, 306, 706, 806) eine Umhüllung (702a, 702b, 702c, 806a, 806b) umfasst, die dazu konfiguriert ist, die Expansionskammer (104, 204, 304, 804) zurückzuhalten, und wobei optional die Umhüllung (702a, 702b, 702c, 806a, 806b) eine obere Schicht und eine untere Schicht umfasst und wobei die obere Schicht und die untere Schicht aneinander befestigt sind, um die Umhüllung zu bilden.

12. Gerät zur Schienung nach Anspruch 10 oder Anspruch 11, wobei die Außenhülse (106, 206, 306, 706, 806):
aus einem flexiblen Material gebildet ist; und/oder
ferner Befestigungselemente (207, 307) zum Befestigen des Geräts zur Schienung um einen Körperteil eines Patienten umfasst; und/oder
so bemessen und geformt ist, dass es eine C-förmige Schiene bildet; und/oder
dazu konfiguriert ist, zusätzlich eine Metallschiene (714) zum Schienen eines relativ langen Körperglieds zu umschließen; und/oder
einen Verbindungsabschnitt (806c) zum Trennen der ersten und zweiten Umhüllung (806a, 806b) umfasst, wobei jede von der ersten und zweiten Umhüllung (806a, 806b) eine Expansionskammer (804) zurückhält.

13. System zur Schienung, das eine Vielzahl von Geräten zur Schienung nach einem der vorhergehenden Ansprüche umfasst.

14. Verfahren zum Aktivieren des Geräts zur Schienung nach einem der Ansprüche 1 bis 12, wobei das Verfahren Ausüben einer Kraft auf das Gerät zur Schienung (100, 200, 300, 400, 700) umfasst, um die Opfernaht (103) aufzubrechen, und wobei optional der Schritt des Ausübens einer Kraft Ausüben einer Druckkraft auf das Gerät zur Schienung (100, 200, 300, 400, 700) umfasst.

15. Verfahren nach Anspruch 14, wobei der Schritt des Ausübens einer Kraft Verdrehen des Geräts zur Schienung (100, 200, 300, 400, 700) umfasst, um eine Torsionskraft auf das Gerät zur Schienung (100, 200, 300, 400, 700) auszuüben, und optional Verdrehen des Geräts zur Schienung (100, 200, 300, 400, 700) im Uhrzeigersinn und gegen den Uhrzeigersinn umfasst.

## Revendications

1. Appareil d'attelle (100, 200, 300, 400, 700) comprenant :
une poche de retenue (102, 202, 302, 402, 704, 802) comportant une première partie (102a, 402a) contenant un premier précurseur, une seconde partie (102b, 402b) contenant un second précurseur, et au moins une couture sacrificielle (103) séparant fluidiquement la première partie (102a, 402a) de la seconde partie (102b, 402b) ; et
une chambre d'expansion (104, 204, 304, 804) entourant la poche de retenue (102, 202, 302, 402, 704, 802) ;
ladite au moins une couture sacrificielle (103) pouvant être rompue lors de l'application d'une force sur la poche de retenue (102, 202, 302, 402, 704, 802) pour ainsi relier fluidiquement les première et seconde parties (102a, 102b, 402a, 402b) de la poche de retenue (102, 202, 302, 402, 704, 802) de sorte que les premier et second précurseurs se mélangent ensemble, et
ledit mélange des premier et second précurseurs formant une mousse polymère expansée durcissable, et
**caractérisé en ce que** la chambre d'expansion (104, 204, 304, 804) comporte un volume qui est d'environ 110 % du volume de la mousse polymère expansée.

2. Appareil d'attelle selon la revendication 1 :
ladite première partie (102a, 402a) et ladite seconde partie (102b, 402b) de la poche de retenue (102, 202, 302, 402, 704, 802) étant couplées ensemble par la couture sacrificielle (103) ; ou
chacune de la première partie (102a, 402a) et de la seconde partie (102b, 402b) comprend l'une de l'au moins une couture sacrificielle (103).

3. Appareil d'attelle selon une quelconque revendication précédente,
lesdits premier et second précurseurs étant miscibles ; et/ou
chacun des premier et second précurseurs étant un précurseur monomère.

4. Appareil d'attelle selon une quelconque revendication précédente, ledit mélange des premier et second précurseurs fournissant une réaction exothermique fournissant de la chaleur comportant une température allant de 20 degrés Celsius à 37 degrés Celsius.

5. Appareil d'attelle selon une quelconque revendication précédente, ladite mousse polymère expansée :
étant conçue pour durcir dans les limites de 1 à 9 minutes suivant la rupture de la couture sacrificielle ; et/ou
étant une mousse à cellules fermées ; et/ou
étant moulable avant le durcissement.

6. Appareil d'attelle selon une quelconque revendication précédente,
ledit volume de la mousse polymère expansée étant d'environ 20 fois le volume total des précurseurs avant le mélange ; et/ou
ladite poche de retenue (102, 202, 302, 402, 704, 802) étant conçue pour éclater lors de l'expansion des premier et second précurseurs lorsqu'ils se mélangent pour former la mousse polymère expansée.

7. Appareil d'attelle selon une quelconque revendication précédente,
chacune des première et seconde parties (102a, 102b, 402a, 402b) de la poche de retenue (102, 202, 302, 402, 704, 802) étant conçue sous forme de tubes allongés ; et/ou
lesdites première et seconde parties (102a, 102b, 402a, 402b) s'étendant le long de 50 % à 90 % de la longueur de la chambre d'expansion (104, 204, 304, 804).

8. Appareil d'attelle selon une quelconque revendication précédente, comprenant en outre au moins une poche de retenue supplémentaire (202).

9. Appareil d'attelle selon une quelconque revendication précédente, ladite chambre d'expansion (104, 204, 304, 804) étant formée sous la forme d'une vessie ou d'un sac.

10. Appareil d'attelle selon une quelconque revendication précédente, comprenant en outre un manchon externe (106, 206, 306, 706, 806) entourant la chambre d'expansion (104, 204, 304, 804), et éventuellement ladite chambre d'expansion (104, 204, 304, 804) étant fixée à une surface interne du manchon externe (106, 206, 306, 706, 806).

11. Appareil d'attelle selon la revendication 10, ledit manchon externe (106, 206, 306, 706, 806) comprenant une enveloppe (702a, 702b, 702c, 806a, 806b) conçue pour retenir la chambre d'expansion (104, 204, 304, 804), et éventuellement ladite enveloppe (702a, 702b, 702c, 806a, 806b) comprenant une couche supérieure et une couche inférieure, et ladite couche supérieure et ladite couche inférieure étant fixées ensemble pour former l'enveloppe.

12. Appareil d'attelle selon la revendication 10 ou la revendication 11, ledit manchon externe (106, 206, 306, 706, 806) :
étant formé à partir d'un matériau souple ; et/ou
comprenant en outre des éléments de fixation (207, 307) pour fixer l'appareil d'attelle autour d'une partie corporelle d'un patient ; et/ou
étant dimensionné et façonné pour former une attelle en forme de C ; et/ou
étant conçu pour enfermer en outre une attelle métallique (714) pour mettre un membre relativement long dans une attelle ; et/ou
comprenant une partie de jonction (806c) destinée à séparer les première et seconde enveloppes (806a, 806b), chacune des première et seconde enveloppes (806a, 806b) retenant une chambre d'expansion (804).

13. Système d'attelle comprenant une pluralité d'appareils d'attelle selon une quelconque revendication précédente.

14. Procédé d'activation de l'appareil d'attelle selon l'une quelconque des revendications 1 à 12, le procédé comprenant l'application d'une force à l'appareil d'attelle (100, 200, 300, 400, 700) pour rompre la couture sacrificielle (103), et éventuellement ladite étape d'application d'une force comprenant l'application d'une force de compression à l'appareil d'attelle (100, 200, 300, 400, 700).

15. Procédé selon la revendication 14, ladite étape d'application d'une force comprenant la torsion de l'appareil d'attelle (100, 200, 300, 400, 700) pour fournir une force de torsion à l'appareil d'attelle (100, 200, 300, 400, 700), et comprenant éventuellement la torsion de l'appareil d'attelle (100, 200, 300, 400, 700) dans le sens horaire et anti-horaire.
